# EUROPÄISCHE PATENTSCHRIFT

(11) **EP 0 941 251 B1**
(45) Veröffentlichungstag und Bekanntmachung des Hinweises auf die Patenterteilung: **04.09.2002**
(21) Anmeldenummer: 97910357.9
(22) Anmeldetag: 25.09.1997
(51) Int. Cl.: C07K 16/12, C07K 16/04, A61K 39/108, A61K 39/40, A23C 9/20

(54) **VERWENDUNG EINES IMMUNGLOBULINPRÄPARATES ZUR HERSTELLUNG EINES ORAL ANZUWENDENDEN MEDIKAMENTS ZUR PRÄVENTION DES HÄMOLYTISCHEN URÄMISCHEN SYNDROMS SOWIE PRÄPARAT HIERFÜR**
USE OF AN IMMUNOGLOBULIN PREPARATION FOR PRODUCING AN ORAL MEDICAMENT PREVENTING HEMOLYTIC UREMIC SYNDROME AND PREPARATION FOR THE SAME
EMPLOI D'UNE PREPARATION D'IMMUNOGLOBULINE POUR FABRIQUER UN MEDICAMENT A USAGE ORAL POUR LA PREVENTION DU SYNDROME HEMOLYTIQUE ET UREMIQUE, AINSI QUE PREPARATION A CET EFFET

(30) Priorität: 09.10.1996 DE 19641466
(43) Veröffentlichungstag der Anmeldung: 15.09.1999
(73) Patentinhaber: Karch, Helge, Dr., 97204 Höchberg (DE); Lissner, Reinhard, 63937 Gönz-Weilbach (DE); Arnold, Alfred, 63110 Rodgau (DE); Möller, Wolfgang, Dipl.-Chem. Dr., 61440 Oberursel (DE); Huppertz, Hans-Iko, 97080 Würzburg (DE)
(72) Erfinder: Karch, Helge, Dr., 97204 Höchberg (DE); Lissner, Reinhard, 63937 Gönz-Weilbach (DE); Arnold, Alfred, 63110 Rodgau (DE); Möller, Wolfgang, Dipl.-Chem. Dr., 61440 Oberursel (DE); Huppertz, Hans-Iko, 97080 Würzburg (DE)
(74) Vertreter: Beil, Hans Christoph, Dr.
(86) Internationale Anmeldenummer: EP9705262
(87) Internationale Veröffentlichungsnummer: WO98015578

(56) Entgegenhaltungen:
- EP-A- 0 102 831
- WO-A-89/10139
- WO-A-95/08562
- WO-A-97/16977
- LISSNER ET AL.: "A STANDARD IMMUNOGLOBULIN PREPARATION PRODUCED FROM BOVINE COLOSTRA SHOWS ANTIBODY REACTIVITY AND NEUTRALIZATION ACTIVITY AGAINST SHIGA-LIKE TOXINS AND EHEC-HEMOLYSIN OF ESCHERICHIA COLI O157:H7" INFECTION, Bd. 24, Nr. 5, 1996, Seiten 378-383, XP002054610
- SCHMIDT ET AL.: "MOLECULAR ANALYSIS OF THE PLASMID-ENCODED HEMOLYSIN OF ESCHERICHIA COLI O157:H7 STRAIN EDL 933" INFECTION AND IMMUNITY, Bd. 63, Nr. 3, 1995, Seiten 1055-1061, XP002054611

## Beschreibung

Die vorliegende Erfindung betrifft gemäß Anspruch 1 die Verwendung eines Immunglobulinpräparates mit hohem Antikörpergehalt gegen EHEC-Hämolysin zur Herstellung eines oral anzuwendenden Medikaments zur Prävention des Hämolytischen Urämischen Syndroms nach EHEC-Infektionen. Die Unteransprüche betreffen bevorzugte Ausgestaltungen der erfindungsgemäßen Anwendung sowie ein Präparat für die obengenannte Anwendung.

Enterohämorrhagische Escherichia Coli (EHEC) werden zunehmend als wichtiger pathogener Erreger im menschlichen Darmtrakt erkannt. Sie sind seit 1982 als Ursache für blutige Durchfälle beim Menschen bekannt. E. coli O157 ist dabei der am häufigsten vorkommende EHEC-Serotyp.

E. coli 0157 wurde bisher weltweit, besonders in den nördlichen Staaten und hier besonders in den wärmeren Monaten des Jahres gefunden. Typische klinische Manifestationen dieser durch EHEC verursachten Gastroenteritis sind Übelkeit, Krämpfe und blutige Durchfälle. Die häufigste Ursache für den Ausbruch von Infektionen sind kontaminiertes Fleisch und unpasteurisierte, rohe Milch. Je mehr Fälle von EHEC-Infektionen bekannt wurden, um so deutlicher wurde, daß E.coli 0157 und andere EHEC-Serotypen als wichtige Ursache für das Auftreten des Hämolytischen Urämischen Syndroms (HUS) verantwortlich sind.

HUS ist ein Krankheitsbild, das durch die Leitsymptome einer akuten hämolytischen Anämie, Thrombozytopenie und Nephropathie gekennzeichnet ist. HUS ist die häufigste Ursache des akuten Nierenversagens im Kindesalter. Es wird aber auch zunehmend bei Erwachsenen und alten Menschen diagnostiziert. Ohne adäquate Behandlung ist die Lethalität hoch. Typischerweise manifestiert sich HUS während und nach einer Gastroenteritis oder einer blutigen Kolitis, erkennbar an einer plötzlichen, oft nach zwischenzeitlicher Besserung, erneuten Verschlechterung des Allgemeinzustandes mit Blasse und Einschränkung der Urinproduktion. Eine schwere Langzeitfolge von HUS sind Nierenkomplikationen, die zum Verlust der Nierenfunktion führen können. Ungefähr 10% der Kinder mit EHEC-assoziierter Diarrhoe entwickeln ein Hämolytische Urämische Snydrom. In ca. 30 % der Fälle führt das HUS zu schwerem Nierenversagen und in rund 10% der Fälle trotz der Fortschritte der supportiven Therapie, wie z.B. der rechtzeitigen Dialyse und einem Plasmaaustausch, zum Tod. Man nimmt heute an, daß ein Großteil der späteren Nierenversagen und daraus resultierende Nierentransplantationen durch ein nicht erkanntes HUS im Kindesalter verursacht werden.

Die meisten von HUS-Patienten gewonnenen EHEC-Isolate bilden anders als alle anderen E. coli-Bakterien große Mengen Bakteriophagen-codierte Verotoxine (Shiga-like Toxin, SLT), z.B. Verotoxin 1 (SLT-I) und Verotoxin 2 (SLT-II). Die Wirkung dieser Bakterien-Toxine ist nicht zu vergleichen mit den klassischen hitzelabilen oder hitzestabilen E. coli-Enterotoxinen oder mit Endotoxin. Besonders das Auftreten von Verotoxin 2 ist häufig assoziiert mit dem Hämolytischen Urämischen Syndrom.

Normalerweise bei einer Enteritis angewandte Therapeutika, wie z.B. Antibiotika, können die Toxinproduktion und die Freisetzung von Verotoxinen von EHEC-Isolaten in vitro und möglicherweise auch in vivo stimulieren. Motilitätshemmende Antidiarrhoika sind ebenfalls kontraindiziert, da sie die Resorption der Toxine aus dem Darm fördern. Dies alles führt zu einer Verschlechterung der Prognose der EHEC-Erkrankung und einem erhöhten Risiko zur Entwicklung eines HUS. Damit versagen die klassischen, bei einer Enteritis angewandten Therapien, und die Entwicklung des HUS läßt sich bisher durch keine wirksame Maßnahme verhindern.

Seit einiger Zeit wird auch die Verwendung von Immunglobulinen zur Prophylaxe bzw. Therapie von HUS vorgeschlagen und erprobt.

In der PCT-Anmeldung WO 89/10139 wird ein Präparat mit Antikörperaktivität aus Kolostralmilch von nicht immunisierten Säugern beschrieben, das gemäß Beispiel 13 auch Antikörper gegen das Verotoxin 1 (Shiga-like Toxin, SLT-I) der EHEC-Bakterien aufweist.

Gibt man peroral ein Kolostralmilchpräparat mit hoher Antikörperaktivität gegen SLT-I Kindern mit einer EHEC-Infektion mit positivem Verotoxin-Nachweis, so kommt es in vivo nicht zu einer nennenswerten Reduktion oder gar Eradikation dieser Toxine im Darm. Auch 21 Tage nach Therapie-Beginn waren in einer kontrollierten Studie in 7 von 10 Patienten immer noch SLT-I im Stuhl nachweisbar.

Von Pirro, F. (Veterinary Microbiology 43, 131-141, 1995) wurde gezeigt, daß Rinderkolostrum und Rinderserum den zytotoxischen Effekt der Verotoxine in vitro neutralisieren kann. Während alle Proben Antikörper gegen SLT-I enthielten, hatten nur 14,7 % neutralisierende Antikörper gegen SLT-II.

In Tierversuchen mit Hasen war humanes Immunglobulin G nicht in der Lage, die SLT-II assoziierten Erkrankungen zu verhindern. Die subkutane Gabe von Human-IgG hatte nur eine Stunde nach der Infektion Erfolg bei Verhinderung des SLT-I verursachten Durchfalls. Nach 6 bzw. 24 Stunden konnte kein Effekt mehr festgestellt werden (Havens, L. et al., Microbiol. Immunol. 36, 1077-1085, 1992).

Auch von Finazzi, G. (American Journal of Hematology 41, 165-169, 1992) wurde an Patienten mit Hamolytisch Urämischem Syndrom gezeigt, daß die Gabe von intravenösen IgG-Präparaten keinen therapeutischen Erfolg zeigte. Als Therapie der Wahl wurde ein kompletter Plasmaaustausch vorgeschlagen.

Bitzan, M. et al. (Infection 21, 140-145, 1993) stellten zusammenfassend fest, daß bei der Mehrzahl der HUS-Patienten von der Gabe derzeit verfügbarer IgG-Präparate ein Verotoxin-spezifischer therapeutischer Effekt nicht zu erwarten ist.

Von S. Ashenazi et al. (Adv.Exp.Med.Biol. 310, 173-177, 1991) wurde beschrieben, daß durch humane Milch bzw. Kolostrum die Adhäsion von E. coli an die Darmwand inhibiert wird. Die Inhibition wurde durch freie Oligosaccharide oder Mannose-Reste von Glycoproteinen bewirkt, nicht jedoch durch Antikörper der Immunglobulinfraktion, da die Immunglobulinfraktion der humanen Milch nur sehr geringe Mengen an Antikorperaktivitat gegen EHEC enthält. Immunglobuline aus humaner Milch oder Kolostrum eignen sich daher nicht für die Therapie von EHEC-Infektionen und zur Prävention des HUS. Andererseits lassen sich die Oligosaccharidfraktion oder Glykolipide aus humaner Milch oder Kolostrum zur Prophylaxe des HUS nicht sinnvoll einsetzen, da die Verfügbarkeit für einen solchen Einsatz absolut unzureichend ist. Die Ergebnisse lassen sich außerdem nicht auf die Milch oder das Kolostrum anderer Säuger übertragen, da die Zusammensetzung der Oligosaccharidfraktion und der Glykoproteine der humanen Milch sich stark von der der Milch anderer Spezies unterscheiden.

Von Beutin, L. (Bundesgesundheitsblatt 10/94, 54) wurden EHEC-Isolate aus Stuhlproben näher charakterisiert. 67 % zeigten eine SLT-II-Bildung, 54 % eine SLT-I-Bildung. Alle 54 Stämme erwiesen sich als hamolysierend, wobei bei 89 % das EHEC-Hämolysin nachweisbar war. Bei E.coli-Bakterien, die nicht zum Serotyp der EHEC gehören, ist der Phenotyp EHEC-Hämolysin-Bildung nur sehr selten vertreten. Dafür werden zunehmend EHEC-Isolate gefunden, die zwar das Merkmal EHEC-Hämolysin, aber keine SLT-I oder SLT-II aufweisen.

Von H.Schmidt et al. (Infection and Immunity 63, 1055-1061, 1995) wurde das Plasmid-codierte Hämolysin des E. coli 0157 beschrieben. Dem EHEC-Hämolysin wurde eine klinische Bedeutsamkeit zugeschrieben, da es in allen getesteten E.coli 0157-Stämmen vorhanden war. Die Hämolysine werden als wichtige Virulenzfaktoren der Bakterien beschrieben, die unter anderem extraintestinale Erkrankungen hervorrufen können und die mit verschiedenen Zellen, wie z.B. Lymphozyten, Granulozyten, Erythrozyten und Nierenzellen reagieren können. Obwohl das EHEC-Hämolysin rund 70% Homologie zum alpha-Hämolysin anderer E.coli-Stämme aufweist, findet keine Kreuzneutralisation des EHEC-Hämolysins durch Antikörper gegen das alpha-Hämolysin statt. Patienten mit Hämolytischen Urämischen Syndromen bilden spezifische Antikörper gegen das EHEC-Hämolysin in ihrem Serum. Diese spezifischen Antikörper ebenso wie die weit verbreiteten Antikörper gegen alpha-Hämolysin, sind aber offensichtlich nicht in der Lage, das HUS zu verhindern oder zu kurieren.

Aufgabe vorliegender Erfindung ist es daher, auf einfache, kostengünstige Weise ein wirksames Präparat zur Prävention des Hämolytischen Urämischen Syndroms nach EHEC-Infektionen bereitzustellen.

Diese Aufgabe wird erfindungsgemäß dadurch gelöst, daß man ein Immunglobulinpräparat mit hohem Antikörpergehalt gegen EHEC-Hämolysin wie in Anspruch 1 beschrieben oral verwendet.

Überraschenderweise wurde nämlich gefunden, daß eine orale Gabe von Antikörpern gegen dieses EHEC-Hämolysin in der Lage ist, die Stuhlfrequenz der Patienten drastisch zu reduzieren und die Entwicklung des HUS zu verhindern. In über 80% der Behandlungen konnte dabei eine Erradikation des Hämolysins und der Hämolysin-Gen-tragenden Bakterien festgestellt werden, so daß das Risiko der Entwicklung eines HUS nach einer EHEC-Infektion drastisch vermindert wird.

Aufgrund der geringen Durchseuchung der Menschen mit EHEC-Bakterien können keine polyvalenten humanen Immunglobulinpräparate mit ausreichend hoher Aktivität gegen EHEC-Hämolysin und gegebenenfalls gegen SLT-II gewonnen werden, und die Immunisierung einer größeren Anzahl von Menschen mit EHEC-Bakterien oder Hämolysin ist unter ethischen Gesichtspunkten bedenklich.

Überraschenderweise findet man jedoch in der Milch bzw. im Kolostrum von Kühen aus ausgewählten Regionen einen wesentlich höheren Gehalt an Antikörperaktivität gegen EHEC-Hämolysin und gegen EHEC-Hämolysin-Gen-tragende Bakterien, die dazu noch in der Lage sind, das Toxin bzw. die Toxin-Gen-tragenden Bakterien zu neutralisieren. In der PCT-Anmeldung WO 89/10139 wird zwar festgestellt, daß Kolostralmilch von nicht immunisierten Säugern Antikörper gegen nahezu das gesamte Spektrum der Toxine darmpathogener coli-Keime enthalte. In den folgenden Jahren hat sich aber herausgestellt, daß in der Kolostralmilch nicht immunisierter Kühe gegen einige Toxine keine Antikörper oder Antikörper nur in der Kolostralmilch weniger Kühe (Beispiel SLT-II) vorhanden sind bzw. selbst hohe in vitro gefundene Antikörpertiter in vivo nicht protektiv waren (Beispiel SLT-I). Umso überraschender war die protektive Wirksamkeit der Immunglobuline mit Antikörperaktivität gegen EHEC-Hämolysin und gegen EHEC-Hämolysin-Gen-tragende Bakterien.

Präparate mit hohem Antikörpertiter gegen EHEC-Hämolysin lassen sich insbesondere durch Analysieren der Einzelspenden von z.B. Kühen und Auswahl solcher Spenden mit einem hohen Antikörpergehalt gegen EHEC-Hämolysin gewinnen. Gegebenenfalls kann man auf dieses Screening der Einzelspender verzichten, wenn man Milch von Kühen aus Herden sammelt, die einen hohen Durchseuchungsgrad mit EHEC-Hämolysin haben und die deshalb hohe natürliche Antikörper-Aktivitäten aufweisen. Alternativ kann man Kühe vor dem Kalben mit EHEC-Hämolysin und gegebenenfalls mit EHEC-Hämolysin-Gen-tragenden Bakterien immunisieren, um eine noch höhere Antikörper-Aktivität in der Kolostralmilch oder der Milch zu erhalten. Unter Kolostralmilch versteht man hierbei die Milch der ersten fünf Tage nach dem Kalben, während die Milch ab dem sechsten Tag nach dem Kalben als Milch bezeichnet wird. Prinzipiell kommen für die Immunisierung als Spendertiere auch andere Milch-gebende Säugetiere in Frage. Hierzu gehören z.B. Ziegen und Schafe.

Ein erfindungsgemäßes Immunglobulinpräparat kann z.B. aus Rinderkolostralmilch hergestellt werden. Hierzu wird Kolostralmilch entfettet und einer Kurzzeiterhitzung für 20 Sekunden bei 72°C unterzogen. Die Kolostralmagermilch wird auf pH 4,4 bis 4,7 eingestellt und das präzipitierte Kasein durch Filtration oder Zentrifugation abgetrennt. Die Kolostralmolke wird durch Ultrafiltration konzentriert und anschließend sterilfiltriert. Verwendet man Kolostralmilch des ersten Gemelkes, enthält das Präparat einen Immunglobulinanteil von 70-80%, bezogen auf das Gesamtprotein. Das Präparat kann flüssig konfektioniert oder sprüh- oder gefriergetrocknet werden. Alternativ kann man aus der Molke von Kuhmilch, wie sie z.B. bei der Käseherstellung anfällt, durch chromatographische Techniken, durch Ultra-/Diafiltration oder durch Fällungsreaktionen die Immunglobulinfraktion isolieren.

Zur Steigerung der Antikörperaktivität gegen EHEC-Hämolysin können die Kühe mit EHEC-Hämolysin, und bevorzugt zusätzlich mit Oberflächen-Antigenen EHEC-Hämolysin-Gen-tragenden Bakterien, und besonders bevorzugt zusätzlich mit SLT-II immunisiert werden. Die Immunisierung erfolgt dabei vorzugsweise mit den inaktivierten Toxinen bzw. Bakterien und kann subkutan in den Euter oder intranasal erfolgen. Zur Verbesserung des Impferfolges wird vorzugsweise ein Adjuvans (z.B.

Titermax, Vertrieb Sa. Serva, Heidelberg) der Vaccine beigemischt.

Das wie oben geschildert oder auf andere bekannte Weise (z.B. aus Eiern von immuniserten Hühnern) gewonnene Immunglobulinpräparat mit hohem Antikörpergehalt gegen EHEC-Hämolysin wird oral in Mengen von 2-50 g/Tag in Form eines Pulvers, Dragees, Tabletten oder aufgelöst in Wasser, Milch oder anderen Fülligkeiten verabreicht.

Vorzugsweise weist das Präparat zusätzlich einen hohen Antikörpergehalt gegen Oberflächenantigene von EHEC-Hämolysin-Gen-tragenden Bakterien auf, der wie oben geschildert erzielt werden kann.

Von Vorteil kann zusätzlich auch ein Antigenkörpergehalt gegen Shiga-like Toxin II (SLT-II) sein.

Insbesondere weist das Präparat folgende Titer auf, bezogen auf eine Immunglobulinlösung mit 5g/100 ml Immunglobulingehalt: Im Immunoblot nach Gunzer et al (J.Clin.Microbiol. 31 (1993), 2604-2610) und Schmidt et al. (Infect.Immun. 63 (1995), 1055-1061) soll der Titer gegen EHEC-Hämolysin reagierende Antikörper ≥ 1:6400 sein. 50% der hämolytischen Aktivität des EHEC-Hämolysins gegen Erythrozyten nach Bauer et al. (Infect.Immun. 64 (1996), 167-175) sollen noch mit einer Verdünnung der Immunglobulin-Lösung ≥ 1:50 neutralisiert werden. Der Titer neutralisierender Antikörper ggen SLT-II, gemessen im Standard-Neutralisationstest gemäß Schmidt et al. (Infect.Immun. 61 (1993), 534-543), soll ≥ 1:800 sein.

Das Präparat ist besonders indiziert bei blutigen, frequenten, mit krampfartigen Bauchschmerzen einhergehenden akuten Durchfallerkrankungen, insbesondere bei Kindern und älteren Menschen mit nachgewiesener oder vermuteter EHEC-Infektion, um die darauffolgende HUS-Entwicklung zu verhindern.

Die Erfindung wird anhand des folgenden Beispiels näher erläutert.

### Beispiel

Von 36 Kindern mit nachgewiesener EHEC-Infektion wurden 18 Kinder 14 Tage lang mit jeweils 3 mal 7 g eines Immunglobulinpräparates aus Kolostrum mit hohem Antikörpergehalt gegen EHEC-Hämolysin und SLT-II behandelt. Die Kontrollgruppe erhielt die gleiche Menge Rinder-Gelatine als Placebo. Die Patienten wurden weitere 7 Tage beobachtet.

Das Immunglobulinpräparat wurde aus Kolostralmilch von Kühen aus einer geschlossenen Herde mit hoher EHEC-Prävalenz gewonnen. Hierzu wurde das erste Gemelk der Kolostralmilch entfettet und einer Kurzzeiterhitzung für 20 Sekunden bei 72°C unterzogen. Die Kolostralmagermilch wurde auf pH 4,5 eingestellt und das präzipitierte Kasein durch Filtration abgetrennt. Die Kolostralmolke wurde durch Ultrafiltration konzentriert, sterilfiltriert und gefriergetrocknet. Das Präparat enthielt einen Immunglobulinanteil von 75 %, bezogen auf das Gesamtprotein. Im Immunoblot ließ sich bis zu einer Verdünnung von 1:12800 gegen EHEC-Hämolysin reagierende Antikörper nachweisen. 50 % der hämolytischen Aktivität des EHEC-Hämolysins gegen Erythrozyten konnte noch mit einer Verdünnung 1:100 neutralisiert werden. Der Gehalt neutralisierender Antikörper gegen SLT-II war noch in einer Verdünnung von 1:1600 nachweisbar.

Nach Beendigung der Studie lag die durchschnittliche Stuhlfrequenz in der Behandlungsgruppe bei 1 Stuhl pro Tag. In der Placebogruppe wurden 3 Stühle pro Tag beobachtet.

In der Behandlungsgruppe wurden EHEC-Hämolysin-Gen-tragende Bakterien nur noch in rund 11 %, in der Placebogruppe dagegen in rund 43 % der untersuchten Patienten gefunden.

In der Behandlungsgruppe wurden nach 21 Tagen keine SLT-II-Gen-tragende Bakterien mehr nachgewiesen, in der Placebogruppe hat man diese dagegen noch in 2 von 5 Patienten beobachtet.

Unter der Behandlung mit dem erfindungsgemäßen Immunglobulinpräparat wurde kein HUS beobachtet. Sowohl die Elimination der EHEC-Hämolysin-Gen-tragenden als auch der SLT-II-Gen-tragenden Bakterien und die damit einhergehende signifikante Reduktion der Stuhlfrequenz senken drastisch das Risiko zur Entwicklung eines HUS.

## Patentansprüche

1. Verwendung von Immunglobulinpräparaten mit einem Antikörpergehalt gegen EHEC-Hämolysin gemäß einem Titer von ≥ 1:6400 einer Lösung von 5 g Immunglobulin pro 100 ml Immunglobulinlösung zur Herstellung eines oral anzuwendenden Medikaments zur Prävention des Hämolytischen Urämischen Syndroms nach EHEC-Infektion.

2. Verwendung von Immunglobulinpraparaten gemäß Anspruch 1, **dadurch gekennzeichnet, daß** das Immunglobulinpraparat zusätzlich einen hohen Antikörpergehalt gegen Oberflächenantigene von EHEC-Hämolysin -Gen-tragenden Bakterien aufweist.

3. Verwendung von Immunglobulinpräparaten gemäß Anspruch 1 oder 2, **dadurch gekennzeichnet, daß** das Immunglobulinpräparat zusätzlich einen Antikörpergehalt gegen Shiga-like Toxin II gemäß einem Titer von ≥ 1:800 einer Lösung von 5 g Immunglobulin pro 100 ml Immunglobulinlösung aufweist.

4. Verwendung von Immunglobulinpräparaten gemäß Anspruch 1 bis 3, **dadurch gekennzeichnet, daß** das Präparat den Antikörpertiter durch Screening von Einzelspendern aufweist.

5. Verwendung von Immunglobulinpräparaten gemäß Anspruch 1 bis 3, **dadurch gekennzeichnet, daß** das Präparat den Antikörpertiter durch Immunisierung der Einzelspender aufweist.

6. Verwendung von Immunglobulinpräparaten gemäß Anspruch 1 bis 5, **dadurch gekennzeichnet, daß** das Immunglobulinpräparat aus der Milch oder Kolostralmilch milchgebender Säugetiere gewonnen wird.

7. Verwendung von Immunglobulinpräparaten gemäß Anspruch 1 bis 6, **dadurch gekennzeichnet, daß** das Immunglobulinpräparat aus der Milch oder Kolostralmilch von Rindern gewonnen wird.

8. Immunglobulinpräparat, erhältlich aus Milch oder Kolostralmilch milchgebender Säuger, **dadurch gekennzeichnet, daß** es einen Antikörpergehalt gegen EHEC-Hämolysin gemäß einem Titer von ≥ 1:6400 einer Lösung von 5 g Immunglobulin pro 100 ml Immunglobulinlösung aufweist.

9. Immunglobulinpräparat nach Anspruch 8, **dadurch gekennzeichnet, daß** es zusätzlich einen hohen Antikörpergehalt gegen Oberflächenantigene von EHEC-Hämolysin-Gen-tragenden Bakterien aufweist.

10. Immunglobulinpräparat nach Anspruch 8 oder 9, **dadurch gekennzeichnet, daß** es aus Milch oder Kolostralmilch von Rindern erhältlich ist.

## Claims

1. Use of immunoglobulin preparations having an antibody content against EHEC haemolysin according to a titre of ≥ 1:6400 of a solution of 5 g of immunoglobulin per 100 ml of immunoglobulin solution, for the preparation of an orally administrable medicament for the prevention of haemolytic uraemic syndrome following EHEC infection.

2. Use of immunoglobulin preparations according to claim 1, **characterised in that** the immunoglobulin preparation additionally has a high antibody content against surface antigens of EHEC-haemolysin-gene-carrying bacteria.

3. Use of immunoglobulin preparations according to claim 1 or 2, **characterised in that** the immunoglobulin preparation additionally has an antibody content against Shiga-like toxin II according to a titre of ≥ 1:800 of a solution of 5 g of immunoglobulin per 100 ml of immunoglobulin solution.

4. Use of immunoglobulin preparations according to claim 1 to 3, **characterised in that** the preparation has the antibody titre by screening of individual donors.

5. Use of immunoglobulin preparations according to claim 1 to 3, **characterised in that** the preparation has the antibody titre by immunisation of individual donors.

6. Use of immunoglobulin preparations according to claim 1 to 5, **characterised in that** the immunoglobulin preparation is obtained from the milk or colostrum of lactating mammals.

7. Use of immunoglobulin preparations according to claim 1 to 6, **characterised in that** the immunoglobulin preparation is obtained from the milk or colostrum of cattle.

8. Immunoglobulin preparation obtainable from milk or colostrum of lactating mammals, **characterised in that** it has an antibody content against EHEC haemolysin according to a titre of ≥ 1:6400 of a solution of 5 g of immunoglobulin per 100 ml of immunoglobulin solution.

9. Immunoglobulin preparation according to claim 8, **characterised in that** it additionally has a high antibody content against surface antigens of EHEC-haemolysin-gene-carrying bacteria.

10. Immunoglobulin preparation according to claim 8 or 9, **characterised in that** it is obtainable from milk or colostrum of cattle.

## Revendications

1. Utilisation de préparations d'immunoglobulines ayant une teneur en anticorps contre l'hémolysine de EHEC selon un titre ≥ 1:6400 d'une solution de 5 g d'immunoglobulines pour 100 ml de solution d'immunoglobulines pour la production d'un médicament à usage oral pour la prévention du syndrome hémolytique et urémique après une infection par EHEC.

2. Utilisation de préparations d'immunoglobulines selon la revendication 1, **caractérisée en ce que** la préparation d'immunoglobulines comporte en outre une haute teneur en anticorps contre des antigènes de surface de bactéries porteuses de gène d'hémolysine de EHEC.

3. Utilisation de préparations d'immunoglobulines selon la revendication 1 ou 2, **caractérisée en ce que** la préparation d'immunoglobulines présente en outre une teneur en anticorps contre la toxine analogue à Shiga II selon un titre ≥ 1:800 d'une solution de 5 g d'immunoglobulines pour 100 ml de solution d'immunoglobulines.

4. Utilisation de préparations d'immunoglobulines selon les revendications 1 à 3, **caractérisée en ce que** la préparation présente le titre d'anticorps par sélection de donneurs individuels.

5. Utilisation de préparations d'immunoglobulines selon les revendications 1 à 3, **caractérisée en ce que** la préparation présente le titre d'anticorps par immunisation de donneurs individuels.

6. Utilisation de préparations d'immunoglobulines selon les revendications 1 à 5, **caractérisée en ce que** la préparation d'immunoglobulines est obtenue à partir du lait ou du colostrum de mammifères produisant du lait.

7. Utilisation de préparations d'immunoglobulines selon les revendications 1 à 6, **caractérisée en ce que** la préparation d'immunoglobulines est obtenue à partir du lait ou du colostrum de vaches.

8. Préparation d'immunoglobulines pouvant être obtenue à partir du lait ou du colostrum de mammifères produisant du lait, **caractérisée en ce qu'**elle présente une teneur en anticorps contre l'hémolysine de EHEC selon un titre ≥ 1:6400 d'une solution de 5 g d'immunoglobulines pour 100 ml de solution d'immunoglobulines.

9. Préparation d'immunoglobulines selon la revendication 8, **caractérisée en ce qu'**elle présente en outre une haute teneur en anticorps contre des antigènes de surface de bactéries porteuses de gène d'hémolysine de EHEC.

10. Préparation d'immunoglobulines selon la revendication 8 ou 9, **caractérisée en ce qu'**elle peut être obtenue à partir du lait ou du colostrum de vaches.
